(19) Europäisches Patentamt / European Patent Office / Office européen des brevets

(11) **EP 2 987 447 B1**

(12) **EUROPEAN PATENT SPECIFICATION**

(45) Date of publication and mention
of the grant of the patent:
**06.11.2019 Bulletin 2019/45**

(21) Application number: **13882096.4**

(22) Date of filing: **23.05.2013**

(51) Int Cl.:
*A61B 1/00* *(2006.01)*    *A61B 5/06* *(2006.01)*
*A61B 5/07* *(2006.01)*    *A61M 25/01* *(2006.01)*

(86) International application number:
**PCT/CN2013/076162**

(87) International publication number:
**WO 2014/169504 (23.10.2014 Gazette 2014/43)**

(54) **DEVICE FOR CONTROLLING MOVEMENT OF CAPSULE ENDOSCOPE IN HUMAN DIGESTIVE TRACT**

VORRICHTUNG ZUR REGELUNG DER BEWEGUNG EINES KAPSELENDOSKOPS IM MENSCHLICHEN VERDAUUNGSTRAKT

DISPOSITIF POUR LE CONTRÔLE DE MOUVEMENT DE CAPSULE ENDOSCOPIQUE DANS L'APPAREIL DIGESTIF HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.04.2013 CN 201310136094**

(43) Date of publication of application:
**24.02.2016 Bulletin 2016/08**

(73) Proprietor: **Ankon Technologies Co. Ltd.**
**Wuhan, Hubei 430075 (CN)**

(72) Inventors:
• **DUAN, Xiaodong**
**Wuhan**
**Hubei 430075 (CN)**
• **ZHANG, Shaobang**
**Wuhan**
**Hubei 430075 (CN)**
• **XIAO, Guohua**
**Wuhan**
**Hubei 430075 (CN)**
• **WANG, Xinhong**
**Wuhan**
**Hubei 430075 (CN)**
• **WANG, Junjie**
**Wuhan**
**Hubei 430075 (CN)**

(74) Representative: **Viering, Jentschura & Partner mbB**
**Patent- und Rechtsanwälte**
**Am Brauhaus 8**
**01099 Dresden (DE)**

(56) References cited:
**WO-A1-2005/122866**    **WO-A1-2009/107892**
**WO-A1-2012/102240**    **CN-A- 101 001 563**
**CN-A- 101 732 026**    **CN-C- 1 287 727**
**KR-B1- 100 735 863**    **US-A1- 2004 064 153**

**Description**

TECHNICAL FIELD

The invention relates to medical apparatus and instruments, specifically refers to a device for controlling movement of a capsule endoscope in human GI track.

BACKGROUND OF THE INVENTION

With the development of large scale integrated circuit technology, MEMS, wireless communications and optical technology, as an effective way for diagnosis for intestinal diseases, capsule endoscope has been researched widely and took a rapid development currently. The first generation study of capsule endoscope has a mature product and deep research basis at home and abroad. M2A produced by Given Imaging company in Israel, Endo Capsule researched and developed by Olympus company in Japan, and products marketed under a Chinese company, Jinshan Science and Technology, all have taken significant market shares in the capsule endoscope marketplace. The current available wireless capsules adopted in the medical field are carried by peristalsis through a human digestive tract, as a result the movement speed, movement direction and capsule location is random, which makes it difficult for medical doctors to collect the relevant information for intestinal tract diagnosis.

If positioning and controlling a capsule endoscope in vivo cannot be achieved, which would cause multiple issues in an examination process of the capsule endoscope. Current existing capsule endoscopes mainly rely on peristalsis and organs contraction to accomplish the capsule movement along a GI track in vivo. Such movement is not only slow, leading to low detection efficiency and potential dead zones in the examination, but also makes examination or operation in specific disease region impossible, as the movement based on peristalsis cannot move the capsule endoscope back and forth to a precise target location for a stable examination or operation, and such movement does not allow control of movement speed and direction, and the posture.

Domestic Jinshan Group has manually controlled an external magnet to accomplish the position or navigation of a capsule endoscope in GI track. The manual control is low cost, but is less precise than a mechanical robot thus less favorable in a routine testing, which prefers artificial intelligence. Furthermore, several scientific research institutions have demonstrated controlling a magnetic capsule endoscope by a strip shaped external magnet. This method is quick and can precisely place the capsule in a direct route, however, because human GI track is not straight but very snaky, it is very difficult to carry out actual positioning of capsule endoscope using such linear magnet in practical clinical setting.

US patent applications US20070221233 , US20100268026 , US20110054255 , and US20110184235 disclose a floating or suspended capsule. As described in these patent applications, a magnetic capsule is suspended by a surrounding liquid. In clinical practice, since the most commonly used liquid is water, the weight of such floated capsule is limited to be under 3g. For a capsule endoscope whose density is more than water, how to realize stable suspension has not been reported in detail.

Document WO 2005/122866 A1 discloses a capsule endoscope control system which can move a capsule endoscope in a human body by a remote control system outside the human body. The capsule endoscope control system taught therein comprises 1) a medical capsule equipped with a permanent magnet; 2) a wireless transmission circuit for transmitting signals to outside of the body; and 3) 2-DOF rotary joint unit for rotating an external permanent magnet in two directions, thereby external permanent magnet applying magnetic forces to the permanent magnets provided in the capsule. Further, capsule endoscope control system in WO 2005/122866 A1 has a distance sensor for measuring a distance between the external permanent magnet and a surface of the human body and the 2-DOF rotary joint unit works with a Cartesian coordinate robot for moving the external permanent magnet. Moreover, a bed supporting the human body, can roll within a certain degree to compensate the movement of the external magnetic outside the human body.

SUMMARY OF THE INVENTION

Technical problems to be solved on the invention is to overcome the existing shortcomings, and to provide a device and method for controlling movement of a capsule endoscope in human GI track, which is capable of precisely producing 5-dimensional moving and rotating magnetic field, generating remote action force to magnetic capsule endoscope, the device and method solve positioning and control problems of capsule endoscope under movement magnetic field.

In order to solve above technical problems, the invention discloses a device according to claim 1.

In above solution, said base is further provided with an X-axis motor driving the X-axis module sliding on the X-axis slide rail. The bottom of said Y-axis slide rail is provided with a Y-axis base fixedly connected with the Y-axis slide rail. Preferably, said Y-axis slide rail is two in number. In this way, X-axis motor drives X-axis module sliding on X-axis slide rail that enable Y-axis slide rail connected with X-axis module to slide therewith, thus to enable Z-axis bracket on Y-axis slide rail also sliding therewith, and thus to control magnetic ball sliding along the direction parallel to X-axis slide rail. Additionally, two Y-axis slide rails are connected with each other, ensuring a more and steady motion during the process of moving.

In above solution, the device further comprises a Y-axis module which is composed of side plates fixed on both sides of Z-axis bracket and a baseplate fixed at the bottom thereof, said side plate is parallel to the X-axis module, and said baseplate is in sliding contact with the Y-axis slide rail, wherein said one of X-axis module is provided with a Y-axis motor which drives the Y-axis module sliding on Y-axis slide rail, the output end of said Y-axis motor is connected with a drive screw rod successively crossing two side plates and screw jointed therewith. In this way, Y-axis motor drives Y-axis module sliding on Y-axis slide rail, enabling Z-axis bracket connected with Y-axis module sliding therewith, thus to control the magnetic ball sliding along the direction parallel to Y-axis slide rail.

In above solution, wherein the number of said Z-axis slide rail and said Z-axis cantilever is two in number and both two are set in parallel. One end of said each Z-axis cantilever is fixedly connected with a Z-axis module. Said two Z-axis modules are respectively sliding-jointed with a Z-axis slide rail. Z-axis motor is set on the Z-axis bracket, and the output end of Z-axis motor is connected with a screw rod which is connected with two Z-axis modules through a transverse connecting rod.

Or, said Z-axis slide rail is four in number and set in parallel, and said Z-axis cantilever is two in number and set in parallel. One end of said Z-axis cantilever is fixedly connected with a Z-axis module whose both ends are respectively sliding-jointed with two Z-axis slide rails. Two Z-axis modules are parallel to the X-axis module. The Z-axis motor is set on Z-axis bracket, and the output end of Z-axis motor is connected with a screw rod which is connected with two Z-axis modules through a transverse connecting rod.

In this way, Z-axis motor drives Z-axis module sliding on Z-axis slide rail, enabling Z-axis bracket connected with Z-axis module sliding therewith, thus to control the magnetic ball sliding along the direction parallel to the Z-axis slide rail.

In above solution, a horizontal motor is connected with the frame via a vertical shaft. In this way, a horizontal motor controls the frame together with the magnetic ball rotating horizontally through a vertical shaft.

In above solution, the interior part of the frame is provided with a horizontal shaft across through magnetic ball along with horizontal axis, and a synchronizing wheel is further installed in the frame. One of the synchronizing wheel is connected with the vertical motor, and the other end is connected with a horizontal shaft. In this way, a vertical motor drives the magnetic ball rotating vertically through operation of the synchronizing wheel.

In above solution, said magnetic ball is a permanent magnet, or electromagnet, or superconducting magnet. In this way, the movement of capsule endoscope for a small magnet is controlled by magnetic field generated by the magnetic ball.

Described but not claimed is a method for controlling movement of a capsule endoscope in human GI track, wherein said method is carried out as following steps:

A) emptying GI track of a subject;

B) letting the subject lie flatly after taking a magnetic capsule endoscope;

C) setting a device for controlling the motion of capsule endoscope in human GI track at external part of the subject, where said device comprises a base, a X-axis slide rail fixed on the base, a Y-axis slide rail vertical to the X-axis slide rail along the horizontal plane, two X-axis modules respectively fixed with both ends of the Y-axis slide rail and jointed with the X-axis slide rail in a sliding way, a Z-axis bracket vertically set on the Y-axis slide rail and jointed with the same in a sliding way, at least two Z-axis slide rails vertically fixed with the Z-axis bracket, a Z-axis cantilever which is parallel to the horizontal plane and whose one end is jointed with the Z-axis slide rail in a sliding way, an frame connected with the other end of Z-axis cantilever, a Z-axis motor driving the Z-axis cantilever sliding on Z-axis slide rail, and an magnetic ball installed in the frame, the frame being provided with a horizontal motor controlling the rotation of magnetic ball on horizontal direction, and a vertical motor controlling the rotation on vertical direction, adjusting the magnetic ball moving along directions of X, Y and Z axis, and horizontal rotation and vertical spinning motions thereof to control the distance between the magnetic ball and capsule endoscope, which enables the capsule endoscope in a suspended state in human GI track;

D) under a suspended state, controlling said magnetic ball to move along with the direction in parallel to X-axis slide rail and/or Y-axis slide rail, which enables the capsule endoscope moving along with the moving direction of said magnetic ball;

E) under a suspended state, controlling said magnetic ball to move along with the direction parallel to the Z-axis slide rail, which enables the capsule endoscope moving opposite to the moving direction of said magnetic ball;

F) being discharged the capsule endoscope with human wastes after the capsule endoscope moving through the human GI track.

In step (C) of the described method, working conditions under X axis, Y axis, Z axis, horizontal rotation and vertical spinning are noninterference, it is not only capable of working alone in different periods successively, but also working at the same time, or working at any compound states.

Beneficial effects of this described method is that: The suspension and position and posture control of a magnetic capsule endoscope in the human GI track is realized by an external magnetic field of a magnetic ball in the present invention, and, a stable suspension system is established through the magnetic field. Capsule endoscope can move in three directions of X, Y and Z axes, and can be deflected to form various observation angles. As the magnetic ball can accurately generate five rotational magnetic fields with free degree during movement, and can generate remote action force for the magnetic capsule endoscope, a method for scanning the surface of stomach is achieved, and problems of positioning and control of the capsule endoscope in a motion magnetic field are solved, which can improve the detected ratio of the human GI track diseases after popularization and application. According to the described method, the motor pattern that the traditional capsule endoscope performs self-creepage just relying on human GI track is eliminated, and the method has the characteristics of accurate positioning, high control speed, and safety and reliability.

BRIEF DESCRIPTION OF THE DRAWINGS

FIG. 1 is a perspective view of a device for controlling movement of capsule endoscope in human GI track;
FIG. 2 is a partially perspective view of capsule endoscope in FIG. 1 for controlling horizontal and vertical rotations;
FIG. 3 is a perspective view of the device and a human, showing that the device controls movement of the capsule endoscope in Human GI track;
FIG. 4 is a schematic diagram for stable suspension of capsule endoscope in the invention;
FIG. 5 is a gravity and magnetic moment changing graph of capsule endoscope while suspending;
FIG. 6 is a schematic diagram of capsule endoscope moving on X-Y axis while suspending;
FIG. 7 is a schematic diagram of capsule endoscope moving on Z axis while suspending;
FIG. 8 is a schematic diagram of capsule endoscope rotating on magnetic field area of the magnetic ball while suspending;
FIG. 9 is a changing graph of horizontal distance deviating initial position and rotation angle for the magnetic ball of capsule endoscope while suspending;
FIG. 10 is a changing graph of angle in the maximum magnetic field intensity point and rotation angle for the magnetic ball;
FIG. 11 is a motion sate diagram of the magnetic ball while changing the angle of capsule endoscope.

DETAILED DESCRIPTION OF THE PREFERRED EMBODIMENTS

Combined with figures and specific embodiment, a further detailed description for the invention is as follows, and the following embodiment is an explanation of this invention but not limited to the same:

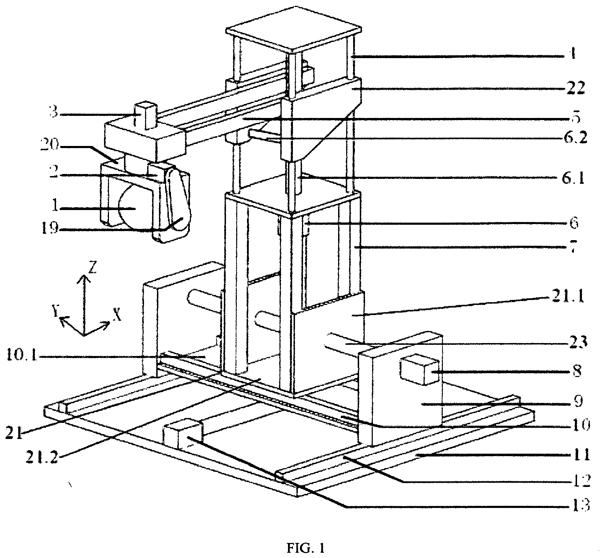

A device for controlling the horizontal and vertical rotations of capsule endoscope shown in FIG. 1 comprises a base 11, two X-axis slide rails 12 fixed on the base 11 in parallel, two Y-axis slide rails 10 vertical to the X-axis slide rail 12 along horizontal plane, two X-axis modules 9 respectively fixed with both ends of the Y-axis slide rail 10 and sliding-joint with the X-axis slide rail 12. The base 11 is further provided with an X-axis motor 13 which drives the X-axis module 9 sliding on the X-axis slide rail 12. Bottom of the Y-axis slide rail 10 is provided with a Y-axis base 10.1 fixedly connected with the same. A Z-axis bracket 7 vertically set on and sliding-jointed with the Y-axis slide rail 10, and the Z-axis bracket 7 is provided with a Y-axis module 21. The Y-axis module 21 is composed of side plates 21.1 fixed on both sides of the Z-axis bracket 7 and a baseplate 21.2 fixed at the bottom thereof. Said side plate 21.1 is parallel to the X-axis module 9, and said baseplate 21.2 is in sliding contact with the Y-axis slide rail 10. Wherein said one of X-axis module 9 is provided with a Y-axis motor 8 which drives the Y-axis module 21 sliding on the Y-axis slide rail 10. Output end of said Y-axis motor 8 is connected with a drive screw rod 23 successively crossing two side plates 21.1 and screw jointed thereof.

Four paralleled Z-axis slide rails 4 are vertically fixed on the Z-axis bracket 7. Two Z-axis cantilevers 5 are provided on the Z-axis bracket 7 and are parallel to the X-axis slide rail 12 along an horizontal direction. One end of each Z-axis cantilever 5 is fixedly connected with a Z-axis module 22, and the other end is connected with an frame 20. Two ends of Z-axis module 22 are respectively jointed with two Z-axis slide rails 4 in a sliding way. Two Z-axis modules 22 are parallel to the X-axis module 9. The Z-axis motor 6 is set on the Z-axis bracket 7. An output end of the Z-axis motor 6 is connected with a screw rod 6.1 that is connected with two Z-axis modules 22 through a transverse connecting rod 6.2. The Z-axis module 22 slides on the Z-axis slide rail (4) through the Z-axis motor (6), thus to drive the Z-axis cantilever (5) sliding up and down.

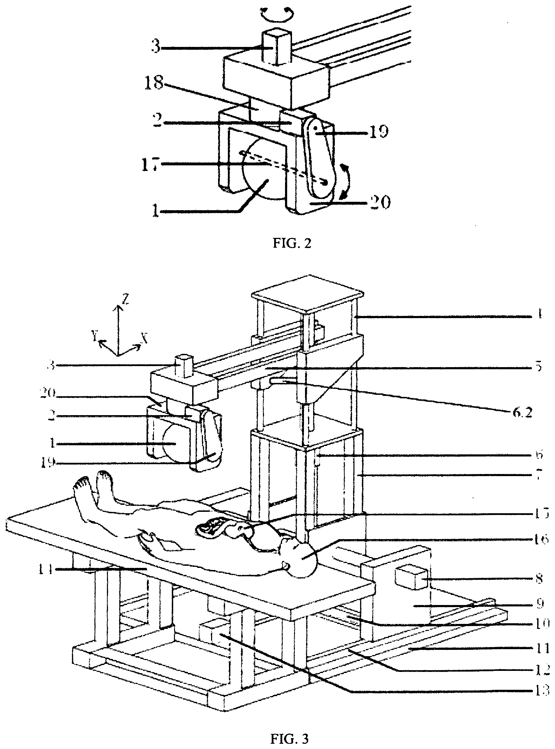

As show in FIG. 2, a magnetic ball 1 installed in frame 20, said magnetic ball 1 is a permanent magnet, or electromagnet, or superconducting magnet. The frame 20 is provided with a horizontal motor 3 controlling the rotation of magnetic ball 1 on horizontal direction, and a vertical motor 2 controlling the rotation of magnetic ball 1 on vertical direction. The horizontal motor 3 is connected with the frame 20 via a vertical shaft 18. Wherein the interior part of the frame 20 is provided with a horizontal shaft 17 across through said magnetic ball 1 along with horizontal axis thereof, and a synchronizing wheel 19 is further installed in the frame 20, one of the synchronizing wheel 19 is connected with a vertical motor 2, the other end thereof is connected with the horizontal shaft 17.

As shown in FIG. 3, a method for controlling movement of a capsule endoscope in human GI track using above device is performed as following steps:

A) abrosia within 4 to 12 hours before experiment, and emptying GI track as much as possible for preventing residues in vivo influencing the shoot of capsule endoscope;

B) letting a subject 16 lie flatly on a berch 14 after taking a capsule endoscope 15 containing magnet;

C) setting said device for controlling the motion of a capsule endoscope 15 in human GI track at external of the subject 16, and the X-axis motor 13, Y-axis motor 8, and Z-axis motor 6 are provided to respectively adjust movements of the X-axis module 9, Y-axis module 21, and Z-axis module 22, which enables the magnetic ball 1 moving along X axis, Y axis and Z axis, and the horizontal motor 3 and vertical motor 2 are controlled to enable the magnetic ball 1 respectively rotating horizontally and vertically, thus to control the distance between said magnetic ball 1 and magnetic capsule endoscope 15, which enables that the total upward buoyancy and attraction from the magnetic ball 1 of the magnetic capsule endoscope 15 is equal to self-gravity thereof, and makes magnetic capsule endoscope 15 be in a suspended state in human GI track;

Working conditions of X-axis motor 13, Y-axis motor 8, Z-axis motor 6, horizontal motor 3 and vertical motor 2 are noninterference, it is not only capable of working alone in different periods successively, but also joint-working of five motors at the same time, or working at any compound state. If five motors work at the same time, X-axis module 9, Y-axis module 21, and Z-axis module 22 slide at the same time, and the frame 20 drives the magnetic ball 1 spinning on horizontal direction, at this time, said magnetic ball 1 also can rotate around the vertical shaft on vertical direction;

D) under a suspended state, controlling X-axis motor 13 and/or Y-axis motor 8 to enable the magnetic ball 1 to move along the direction parallel to the X-axis slide rail 12 and/or Y-axis slide rail 10, namely, the magnetic ball 1 moves along the X-axis or Y-axis, due to receiving gradually enhanced magnetic field force on corresponding direction, and the capsule endoscope 15 moves along with the moving direction of the magnetic ball 1;

E) under a suspended state, controlling said Z-axis motor 6 and the Z-axis module 22 connected with the Z-axis cantilever 5 to slide up and down on the Z-axis slide rail 4, then the magnetic ball 1 also sliding up and down along Z-axis, and the capsule endoscope 15 also moving up and down caused by the enhanced-adverse magnetic field force.

F) based on above steps of said method, controlling the capsule endoscope 15 to move through a certain or whole human GI track, and then discharged with human wastes.

The above contents are only preferable embodiments of the invention, but not used to limit this invention, although the invention is specified in detail referring to aforesaid embodiment, technicians of this field still can modify the technical solution recorded in each aforesaid embedment, or replace partial technical features and the like therein. Any replacement, improvement and the like made within spirits and principles of the invention shall be included in protection scope of the invention.

Stable suspension experiments

The invention further implements a stable suspension experiment on aforesaid device, verifying the feasibility of the invention for controlling movement of a capsule endoscope in human GI track.

Said capsule endoscope 15 of the following experiments comprises magnet.

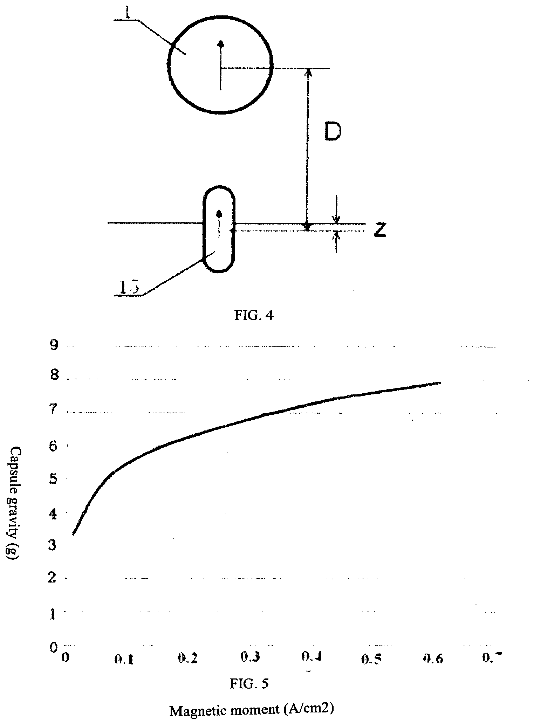

When gravity density is more than liquid density, capsule endoscope 15 performs stable self-suspension relying on external magnetic force, as shown in FIG. 4. Gravity and magnetic moment changing curve of capsule endoscope is as shown in FIG. 5 while suspension.

A capsule endoscope 15 is in suspension under the action of magnetic force:

$$F_m + F_{float} = W$$

Where, W is gravity of capsule endoscope 15, Fm is magnetic force stressed on capsule endoscope 15, $F_{float}$ is buoyancy stressed on capsule endoscope 15.

$$F_m = \frac{\mu_0}{4\pi} \frac{6Mm}{(D+z)^4} \qquad F_{float} = \rho_{liquid}(L/2+z)\pi R^2$$

Where, M is the magnetic moment of the magnetic ball 1 under action of magnetic force; m is magnetic moment of the magnet in the capsule endoscope 15 under action of magnetic force; as shown in FIG. 4, D is the distance from magnetic ball 1 to capsule endoscope 15, z is the distance between gas-liquid interface of human GI track and centre of the capsule endoscope 15, L is the length of capsule endoscope 15,, R is radius of capsule endoscope 15, $\rho_{liquid}$ is a liquid density in Human GI track, $\mu_0$ is the permeability of vacuum.

For the stable suspension of a magnetic capsule, following conditions shall be satisfied:

$$\frac{\partial(W - F_m - F_{float})}{\partial r} = \frac{\mu_0}{\pi} \frac{6Mm}{(D-L/2)^5} - \rho_{liquid}\pi R^2 < 0$$

$$D/L > 4\rho_{capsule}/\rho_{fluid} + 1/2$$

$\rho_{fluid}$ is a liquid density in Human GI track, $\rho_{capsule}$ is a density of capsule endoscope 15;

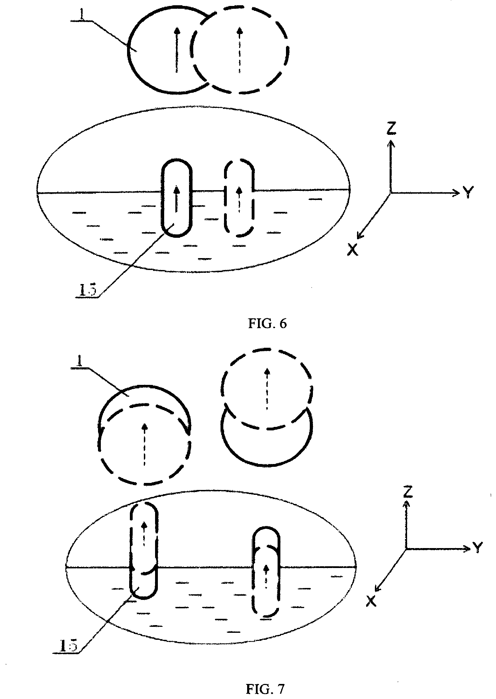

Precise movements of the capsule endoscope 15 on X, Y, and Z axis are controlled by the magnetic ball 1, as shown in FIG. 6 and FIG. 7.

Under a condition of suspension, as shown in FIG. 6, when the magnetic ball 1 is moved on X and Y axis, the capsule endoscope 15 in vivo also moves on X and Y axis horizontally.

Under a condition of suspension, as shown in FIG. 7, when the magnetic ball 1 is moved on Z axis, and when the magnetic ball 1 moves downwards, the capsule endoscope 15 in vivo moves upwards; and when magnetic ball 1 moves upwards, the capsule endoscope 15 in vivo moves downwards. Movements of the capsule endoscope 15 in vivo on Z axis can be precisely controlled via controlling the movement of the magnetic ball 1 on Z axis direction. In this way, the top or bottom of gastric mucosa can be observed in long distance or close range.

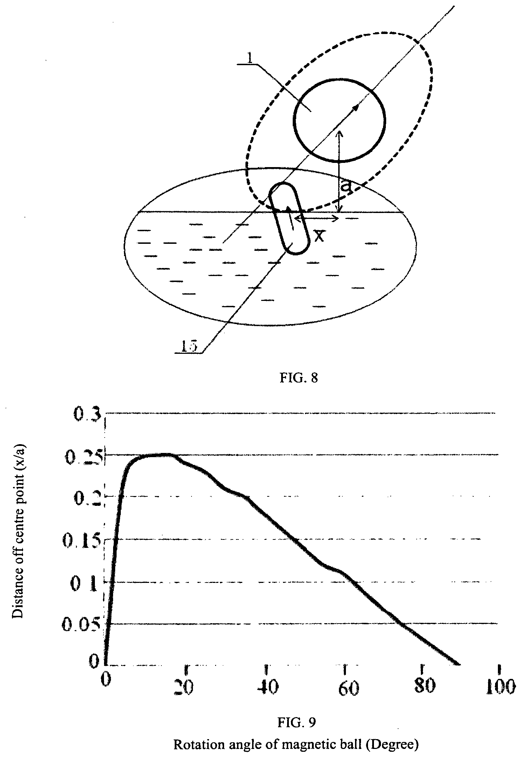

Suspension and rotation methods of capsule endoscope 15 on a particular point are as shown in FIG. 8 to 11, a capsule endoscope 15 is placed with an inclined way on a particular point of the gas-liquid interface in human GI track, the magnetic ball 1 moves a distance on opposite direction to make capsule endoscope 15 adjusted with acting force on Z axis direction. In this way, the capsule endoscope 15 can adjust own angles in any given point. This is the method of capsule endoscope 15 for suspension-inspecting stomach surface.

Under a condition of suspension, as shown in FIG. 8, when magnetic ball 1 rotates towards a same direction, capsule endoscope in vivo also rotates therewith. However, if the maximum magnetic-field location changes, the capsule endoscope in vivo posses a horizontal offset distance from the original location thereof. The dashed ellipse in FIG. 8 is the magnetic equipotential line, the point of contact of the dashed ellipse with gas-liquid interface in human GI track is the maximum point of magnetic field intensity, and the changing curve of the horizontal distance deviating original distance and the rotation angle of the magnetic balll are as shown in FIG. 9.

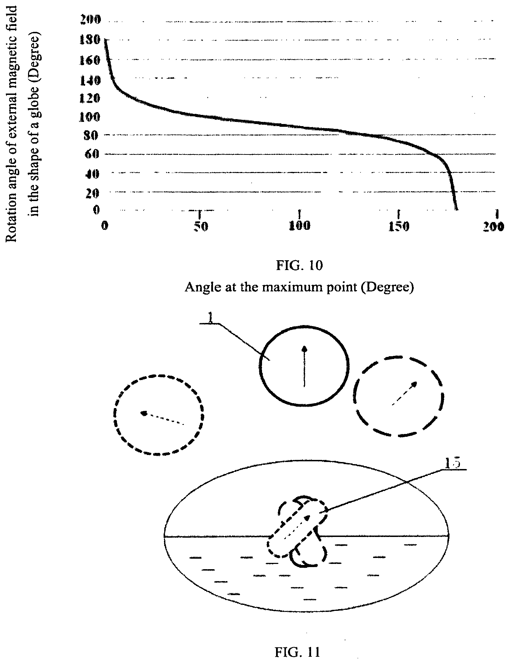

FIG. 10 shows a changing curve of angle in the maximum magnetic field intensity point and rotation angle for the magnetic ball. Rotation angles of the magnetic ball 1 are changed to control inclined angles of capsule endoscope 15. Additionally, FIG. 10 further shows that inclined angles of the capsule endoscope 15 within 45 degrees to 135 degrees are prone to be controlled.

As shown in FIG. 11, when angles of capsule endoscope 15 are changed, the magnetic ball 1 self-rotates and moves along the section of XYZ axis to compensate the distance of moving as shown in FIG. 9, therefore, the capsule endoscope 15 moves and rotates nearby, and in this way, it is beneficial for observing specific conditions around mucosa of GI track.

## Claims

1. A device for controlling movement of a capsule endoscope in a human GI track, wherein the device comprises a base (11), two X-axis slide rails (12) fixed on the base (11) in parallel, at least one Y-axis slide rail (10) vertical to the X-axis slide rail (12) along horizontal plane, two X-axis modules (9) respectively fixed with both ends of Y-axis slide rail (10) and sliding-joint with X-axis slide rail (12), a Z-axis bracket (7) vertically set on and sliding-jointed with Y-axis slide rail (10), a Z-axis slide rail (4) vertically fixed on the Z-axis bracket (7), a Z-axis cantilever (5) which is parallel to horizontal plane and whose one end is sliding-jointed with the Z-axis slide rail (4), a frame (20) connected with the other end of the Z-axis cantilever (5), a Z-axis motor (6) driving the Z-axis cantilever (5) sliding on the Z-axis slide rail (4), and a magnetic ball (1) installed in the frame (20), wherein the frame (20) is provided with a horizontal motor (3) controlling rotation of the magnetic ball (1) in a horizontal plane, and a vertical motor (2) controlling rotation of the magnetic ball (1) in a vertical plane.

2. Device for controlling movement of a capsule endoscope in human GI track according to claim 1, wherein the base (11) is further provided with an X-axis motor (13) which drives the X-axis module (9) sliding on the X-axis slide rail (12), bottom of the Y-axis slide rail (10) is provided with a Y-axis base (10.1) fixedly connected therewith.

3. Device for controlling movement of a capsule endoscope in human GI track according to claim 1, wherein said Y-axis slide rail (10) is two in number.

4. Device for controlling movement of a capsule endoscope in human GI track according to claim 1, wherein the device further comprises a Y-axis module (21) which is composed of side plates (21.1) fixed on both sides of the Z-axis bracket (7) and a baseplate (21.2) fixed at the bottom thereof, said side plate (21.1) being parallel to the X-axis module (9), and said baseplate (21.2) being in sliding contact with the Y-axis slide rail (10), wherein said one of X-axis module (9) is provided with a Y-axis motor (8) which drives the Y-axis module (21) sliding on the Y-axis slide rail (10), and an output end of said Y-axis motor (8) is connected with a drive screw rod (23) successively crossing two side plates (21.1) and screw jointed thereof.

5. Device for controlling movement of a capsule endoscope in human GI track according to claim 1, wherein the number of said Z-axis slide rail (4) and said Z-axis cantilever (5) is two in number respectively and the both of them are set in parallel, one end of said each Z-axis cantilever (5) being fixedly connected with a Z-axis module (22), said two Z-axis modules (22) being respectively sliding-jointed with a Z-axis slide rail (4), the Z-axis motor (6) being set on the Z-axis bracket (7), and the output end of Z-axis motor (6) being connected with a screw rod (6.1) connected with two Z-axis modules (22) through a transverse connecting rod (6.2).

6. Device for controlling movement of a capsule endoscope in human GI track according to claim 1, wherein said Z-axis slide rail (4) is four in number and set in parallel, and said Z-axis cantilever (5) is two in number and set in parallel, one end of said Z-axis cantilever (5) being fixedly connected with a Z-axis module (22) whose both ends are respectively sliding-jointed with two Z-axis slide rails (4), the two said Z-axis modules (22) being parallel to the X-axis module (9), said Z-axis motor (6) being set on the Z-axis bracket (7), and the output end of Z-axis motor (6) being connected with a screw rod (6.1) which is connected with two Z-axis modules (22) through a transverse connecting rod (6.2).

7. Device for controlling movement of a capsule endoscope in human GI track according to claim 1, wherein said horizontal motor (3) is connected with the frame (20) via a vertical shaft (18).

8. Device for controlling movement of a capsule endoscope in human GI track according to claim 1, wherein the interior part of the frame (20) is provided with a horizontal shaft (17) across through the magnetic ball (1) along with horizontal axis thereof, and a synchronizing wheel (19) is further installed in the frame (20), one end of the synchronizing wheel (19) being connected with the vertical motor (2), the other end thereof being connected with the horizontal shaft (17).

9. Device for controlling movement of a capsule endoscope in human GI track according to claim 1, wherein said magnetic ball (1) is a permanent magnet, or electromagnet, or superconducting magnet.

**Patentansprüche**

1. Eine Vorrichtung zum Steuern der Bewegung eines Kapselendoskops in einem menschlichen Gastrointestinaltrakt, wobei
die Vorrichtung aufweist: eine Basis (11), zwei parallel auf der Basis (11) befestigte X-Achsen-Gleitschienen (12), mindestens eine Y-Achsen-Gleitschiene (10) senkrecht zu der X-Achsen-Gleitschiene (12) entlang der horizontalen Ebene, zwei X-Achsen-Module (9), die jeweils an beiden Enden der Y-Achsen-Gleitschiene (10) befestigt und mit der X-Achsen-Gleitschiene (12) gleitverbunden sind, eine Z-Achsen-Halterung (7), die vertikal auf die Y-Achsen-Gleitschiene (10) aufgesetzt und mit dieser gleitverbunden ist, eine Z-Achsen-Gleitschiene (4), die vertikal auf der Z-Achsen-Halterung (7) befestigt ist, einen Z-Achsen-Kragarm (5), der parallel zu der horizontalen Ebene ist und dessen eines Ende mit der Z-Achsen-Gleitschiene (4) gleitverbunden ist, einen Rahmen (20), der mit dem anderen Ende des Z-Achsen-Kragarms (5) verbunden ist, einen Z-Achsen-Motor (6), der den Z-Achsen-Kragarm (5) antreibt, der auf der Z-Achsen-Gleitschiene (4) gleitet, und eine Magnetkugel (1), die in dem Rahmen (20) installiert ist, wobei der Rahmen (20) mit einem horizontalen Motor (3), der das Drehen der Magnetkugel (1) in einer horizontalen Ebene steuert, und mit einem vertikalen Motor (2) versehen ist, der das Drehen der Magnetkugel (1) in einer vertikalen Ebene steuert.

2. Vorrichtung zum Steuern der Bewegung eines Kapselendoskops im menschlichen Gastrointestinaltrakt gemäß Anspruch 1, wobei die Basis (11) ferner mit einem X-Achsen-Motor (13) versehen ist, der das X-Achsen-Modul (9) antreibt, das auf der X-Achsen-Gleitschiene (12) gleitet, wobei der Boden der Y-Achsen-Gleitschiene (10) Y-Achsen-Basis (10.1) versehen ist, die fest damit verbunden ist.

3. Vorrichtung zum Steuern der Bewegung eines Kapselendoskops im menschlichen Gastrointestinaltrakt gemäß Anspruch 1, wobei die Y-Achsen-Gleitschienen- (10) -Anzahl zwei beträgt.

4. Vorrichtung zum Steuern der Bewegung eines Kapselendoskops im menschlichen Gastrointestinaltrakt gemäß Anspruch 1, wobei die Vorrichtung ferner ein Y-Achsen-Modul (21) aufweist, das aus Seitenplatten (21.1), die an beiden Seiten der Z-Achsen-Halterung (7) befestigt sind, und einer Basisplatte (21.2), die am Boden davon befestigt ist, zusammengesetzt ist, wobei die Seitenplatte (21.1) parallel zu dem X-Achsen-Modul (9) ist und die Basisplatte (21) in Gleitkontakt mit der Y-Achsen-Gleitschiene (10) ist, wobei das eine von dem X-Achsen-Modul (9) mit einem Y-Achsen-Motor (8) versehen ist, der das Y-Achsen-Modul (21) antreibt, das auf der Y-Achsen-Gleitschiene (10) gleitet, und ein Ausgangsende des Y-Achsen-Motors (8) mit einer Antriebsspindelstange (23) verbunden ist, die nacheinander zwei Seitenplatten (21.1) kreuzt und damit schraubverbunden ist.

5. Vorrichtung zum Steuern der Bewegung eines Kapselendoskops im menschlichen Gastrointestinaltrakt gemäß Anspruch 1, wobei die Anzahl der Z-Achsen-Gleitschiene (4) und des Z-Achsen-Kragarms (5) jeweils zwei beträgt und die beiden parallel gesetzt sind, wobei ein Ende von jedem Z-Achsen-Kragarm (5) fest mit einem Z-Achsen-Modul (22) verbunden ist, wobei die beiden Z-Achsen-Module (22) jeweils mit einer Z-Achsen-Gleitschiene (4) gleitverbunden sind, wobei der Z-Achsen-Motor (6) auf die Z-Achsen-Halterung (7) gesetzt ist und das Ausgangsende des Z-Achsen-Motors (6) mit einer Spindelstange (6.1) verbunden ist, die über eine Querverbindungsstange (6.2) mit zwei Z-Achsen-Modulen (22) verbunden ist.

6. Vorrichtung zum Steuern der Bewegung eines Kapselendoskops im menschlichen Gastrointestinaltrakt gemäß Anspruch 1, wobei die Z-Achsen-Gleitschienen- (4) - Anzahl vier beträgt und diese parallel gesetzt sind, und wobei die Z-Achsen-Kragarm- (5) -Anzahl zwei beträgt und diese parallel gesetzt sind, wobei ein Ende des Z-Achsen-Kragarms (5) fest mit einem Z-Achsen-Modul (22) verbunden ist, dessen beide Enden jeweils mit zwei Z-Achsen-Gleitschienen (4) gleitverbunden sind, wobei die beiden Z-Achsen-Module (22) parallel zu dem X-Achsen-Modul (9) sind, wobei der Z-Achsen-Motor (6) auf die Z-Achsen-Halterung (7) gesetzt ist und das Ausgangsende des Z-Achsen-Motors (6) mit einer Spindelstange (6.1) verbunden ist, die über eine Querverbindungsstange (6.2) mit zwei Z-Achsen-Modulen (22) verbunden ist.

7. Vorrichtung zum Steuern der Bewegung eines Kapselendoskops im menschlichen Gastrointestinaltrakt gemäß Anspruch 1, wobei der horizontale Motor (3) über eine vertikale Welle (18) mit dem Rahmen (20) verbunden ist.

8. Vorrichtung zum Steuern der Bewegung eines Kapselendoskops im menschlichen Gastrointestinaltrakt gemäß Anspruch 1, wobei der innere Teil des Rahmens (20) mit einer horizontalen Welle (17) quer durch die Magnetkugel (1) zusammen mit der horizontaler Achse davon versehen ist, und wobei ferner ein Synchronisationsrad (19) im Rahmen (20) installiert ist, wobei ein Ende des Synchronisationsrades (19) mit dem vertikalen Motor (2) verbunden ist, wobei das andere Ende davon mit der horizontalen Welle (17) verbunden ist.

9. Vorrichtung zum Steuern der Bewegung eines Kapselendoskops im menschlichen Gastrointestinaltrakt gemäß Anspruch 1, wobei die Magnetkugel (1) ein Permanentmagnet oder Elektromagnet oder supraleitender Magnet ist.

**Revendications**

1. Dispositif pour contrôler le mouvement d'une capsule endoscopique d'un tractus GI (gastro-intestinal) humain, dans lequel :

le dispositif comprend une base (11), deux rails de coulissement d'axe X (12) fixés sur la base (11) en parallèle, au moins un rail de coulissement d'axe Y (10) vertical par rapport au rail de coulissement d'axe X (12) le long du plan horizontal, deux modules d'axe X (9) respectivement fixés avec deux extrémités du rail de coulissement d'axe Y (10) et articulés par coulissement avec le rail de coulissement d'axe X (12), un support d'axe Z placé verticalement sur et articulé par coulissement avec le rail de coulissement d'axe Y (10), un rail de coulissement d'axe Z (4) verticalement fixé sur le support d'axe Z (7), un porte-à-faux d'axe Z (5) qui est parallèle à un plan horizontal et dont une extrémité est articulée par coulissement avec le rail de coulissement d'axe Z (4), un bâti (20) raccordé avec l'autre extrémité du porte-à-faux d'axe Z (5), un moteur d'axe Z (6) entraînant le porte-à-faux d'axe Z (5) coulissant sur le rail de coulissement d'axe Z (4), et une bille magnétique (1) installée dans le bâti (20), dans lequel le bâti (20) est prévu avec un moteur horizontal (3) contrôlant la rotation de la bille magnétique (1) dans un plan horizontal, et un moteur vertical (2) contrôlant la rotation de la bille magnétique (1) dans un plan vertical.

2. Dispositif pour contrôler le mouvement d'une capsule endoscopique dans un tractus GI humain selon la revendication 1, dans lequel la base (11) est en outre prévue avec un moteur d'axe X (13) qui entraîne le module d'axe X (9) coulissant sur le rail de coulissement d'axe X (12), le fond du rail de coulissement d'axe Y (10) est prévu avec une base d'axe Y (10.1) raccordée de manière fixe avec ce dernier.

3. Dispositif pour contrôler le mouvement d'une capsule endoscopique dans un tractus GI humain selon la revendication 1, dans lequel ledit rail de coulissement d'axe Y (10) est au nombre de deux.

4. Dispositif pour contrôler le mouvement d'une capsule endoscopique dans un tractus GI humain selon la revendication 1, dans lequel le dispositif comprend en outre un module d'axe Y (21) qui est composé de plaques latérales (21.1) fixées des deux côtés du support d'axe Z (7) et d'une plaque de base (21.2) fixée sur son fond, ladite plaque latérale (21.1) étant parallèle au module d'axe X (9), et ladite plaque de base (21.2) étant en contact coulissant avec le rail de coulissement d'axe Y (10), dans lequel ledit un des modules d'axe X (9) est prévu avec un moteur d'axe Y (8) qui entraîne le module d'axe Y (21) coulissant sur le rail de coulissement d'axe Y (10) et une extrémité de sortie dudit moteur d'axe Y (8) est raccordée à une tige de vis d'entraînement (23) croisant successivement deux plaques latérales (21.1) et son joint à vis.

5. Dispositif pour contrôler le mouvement d'une capsule endoscopique dans un tractus GI humain selon la revendication 1, dans lequel le nombre dudit rail de coulissement d'axe Z (4) et dudit porte-à-faux d'axe Z (5) est respectivement de deux et les deux sont placés en parallèle, une extrémité dudit porte-à-faux d'axe Z (5) étant raccordée de manière fixe avec un module d'axe Z (22), lesdits deux modules d'axe Z (22) étant respectivement articulés par coulissement avec un rail de coulissement d'axe Z (4), le moteur d'axe Z (6) étant placé sur le support d'axe Z (7), et l'extrémité de sortie du moteur d'axe Z (6) étant raccordée avec une tige de vis (6.1) raccordée avec deux modules d'axe Z (22) par le biais d'une tige de raccordement transversale (6.2).

6. Dispositif pour contrôler le mouvement d'une capsule endoscopique dans un tractus GI humain selon la revendication 1, dans lequel ledit rail de coulissement d'axe Z (4) est au nombre de quatre et placé en parallèle, et ledit porte-à-faux d'axe Z (5) est au nombre de deux et placé en parallèle, une extrémité dudit porte-à-faux d'axe Z (5) étant raccordée de manière fixe avec un module d'axe Z (22) dont les deux extrémités sont respectivement articulées par coulissement avec deux rails de coulissement d'axe Z (4), lesdits deux modules d'axe Z (22) étant parallèles au module d'axe X (9), ledit moteur d'axe Z (6) étant placé sur le support d'axe Z (7), et l'extrémité de sortie du moteur d'axe Z (6) étant raccordée avec une tige de vis (6.1) avec deux modules d'axe Z (22) par le biais d'une tige de raccordement transversale (6.2).

7. Dispositif pour contrôler le mouvement d'une capsule endoscopique dans un tractus GI humain selon la revendication 1, dans lequel ledit moteur horizontal (3) est raccordé avec le bâti (20) via un arbre vertical (18).

8. Dispositif pour contrôler le mouvement d'une capsule endoscopique dans un tractus GI humain selon la revendication 1, dans lequel la partie intérieure du bâti (20) est prévue avec un arbre horizontal (17) sur la bille magnétique (1) conjointement à son axe horizontal, et une roue de synchronisation (19) est en outre installée dans le bâti (20), une extrémité de la roue de synchronisation (19) étant raccordée avec le moteur vertical (2), son autre extrémité étant raccordée avec l'arbre horizontal (17).

**9.** Dispositif pour contrôler le mouvement d'une capsule endoscopique dans un tractus GI humain selon la revendication 1, dans lequel ladite bille magnétique (1) est un aimant permanent ou un électroaimant ou un aimant supraconducteur.

FIG. 1

FIG. 2

FIG. 3

FIG. 4

FIG. 5

Magnetic moment (A/cm2)

FIG. 6

FIG. 7

FIG. 8

FIG. 9

Rotation angle of magnetic ball (Degree)

FIG. 10

Angle at the maximum point (Degree)

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070221233 A
- US 20100268026 A
- US 20110054255 A
- US 20110184235 A
- WO 2005122866 A1